# EUROPEAN PATENT APPLICATION

(11) **EP 3 305 334 A1**
(43) Date of publication of application: **11.04.2018**
(21) Application number: 16192605.0
(22) Date of filing: 06.10.2016
(51) Int. Cl.: A61L 15/18, A61L 15/42

(54) **MAGNETIC COMPOSITION AND MEDICAL MATERIAL FOR STIMULATING VASCULARISATION IN INTERNAL OR EXTERNAL WOUND OF THE BODY**

(71) Applicant: Universitätsklinikum Jena, 07743 Jena (DE)
(72) Inventor: HILGER, Ingrid, 07743 Jena (DE)
(74) Representative: Heinz, Benjamin

(57) **Abstract**

The present invention relates to composition for use in a method for wound treatment, particularly by magnetic hyperthermia, characterized in that said composition comprises a plurality of biocompatible magnetic particles. The present invention further relates to a Medical material for wound treatment comprising a carrier and the composition of the invention.

## Description

The present invention relates to a composition comprising magnetic particles for wound treatment and a medical material comprising the composition.

The healing process of wounds depends on the presence of several local factors such as, for example, systemic mediators, the underlying disease, the kind of injury etc. Those factors determine whether an acute wound healing commences or an abnormal healing process (chronic wound healing).

Chronic wound healing results from an inadequate healing process, in which the anatomic and functional integrity of the tissue is not restored in an appropriate time frame. Approx. 1 % of the European population suffer from chronic wounds. They have an undesirable impact of the life quality and cause considerable economic burdens; 2 % of the healthcare budget is spent on the treatment and care of chronic wounds.

The formation of new blood vessels, the neoangiogenesis, is one of the most important processes in wound healing, and absolutely mandatory for an adequate healing process. However, in chronic wounds the physiological triggering of this process is disturbed. In this context, heat can have a favourable effect on the vascularisation. This process is insofar important in chronic wounds, since there the vessel system is developed only to a minor extent.

Current solutions for promoting wound healing base on the use of intelligent wound dressings, which promote wound healing through heat, growth factors or through their antiseptic properties:
DE 203142527 U1 discloses an electrically heatable skin dressing comprising a flexible carrier layer, an electrical heating means and a skin dressing, wherein the heating means is connected to a source of electricity. However, such electrical heating means bears the danger of short-circuits. Additionally, such skin dressings are limited to external or surface regions of the body.

EP 1782767 A1 discloses a bandage, particularly for treating back problems, providing heat by the exothermic oxidation of iron. Disadvantages of the approach are the relatively long start-up period until the desired temperature is reached (approx. 30 min) and the uncontrollable heat development once the oxidations has started. Also, the bandage can only be used directly after opening of the sterile sealing.

Also known are wound dressings comprising active substances such as growth factors or antimicrobial agents such as silver nanoparticles, which may be also releasable from the wound dressing. However, an active substance released to the wound may be still active when the dressing is removed from the wound, and may cause unwanted side effects.

Known wound-dressing-free means for heat delivery used, for example, in plastic surgery or tumour therapy include radiation with laser light, microwaves, ultrasound, infrared light and radio waves. Disadvantageously, the aforementioned electromagnetic or sound waves are generated in an external source and have to be projected from the source to the target tissue in a focussed and accurate manner. However, focusing techniques can be elaborately and/or inaccurate, and the temperature development may be hardly controllable. In addition, those methods mostly require the use of probes, which have to be inserted into the target tissue and afterwards removed from the tissue limiting their application to open wounds (e.g. during surgery) only. Additionally, the use of radio waves might be accompanied by the generation of undesired electrical fields that may affect the function or integrity of proteins. Likewise, the radiation with infrared light is limited by a relatively poor permeability of tissues for infrared light. Generally, repeating treatments with the aforementioned methods is not or only possible under considerable efforts.

Accordingly, improved means and method for wound healing or wound treatment, particularly of chronic wounds, are still desirable.

It is therefore the objective of the present invention to provide means for deliver heat to wounds for treatment in a simple and efficient way.

This objective is attained by the subject matter of the independent claims. Preferred embodiments of the invention are further specified in the dependent claims described below.

During to course of preclinical experiments for evaluating the use of hyperthermia for tumour therapy, the inventors have surprisingly found that heat applied to the tumour improves or enhances the vascularisation of the affected tissue under certain circumstances, wherein heat advantageously can be generated by magnetic particles in an alternating magnetic field.

According to a first aspect of the invention, a composition for use in a method for wound treatment or wound healing, particularly of a chronic wound, is provided. The composition comprises a plurality of biocompatible magnetic particles, wherein particularly the magnetic particles are configured to generate heat upon application of an alternating magnetic field. Particularly, the magnetic particles may exhibit a magnetic hysteresis when affected by an alternating magnetic field. Furthermore, the magnetic particles may characterized by a single magnetic domain.

The composition of the invention is characterized by several advantages for wound treatment over known means for wound treatment:
- the composition can be delivered directly to the wound to be treated, where heat can be locally generated by application of an alternating magnetic field;
- no undesired side effects occur;
- the heat generation, the heat amount and the duration of heat exposure can effectively and accurately be controlled by control of the alternating magnetic field;
- the heat generation and exposure is not limited by depth of the wound such, for example, in case of exposure of electromagnetic radiation for heat generation;
- heat can be generated multiple times by the composition of the invention by repeating the application of the alternating magnetic field;
- heat can be generated contact-free with the composition of the invention, e.g. without surgical intervention;
- by the composition of the invention the occurrence of undesired short-circuits with body fluids can avoided in comparison to methods that comprises electrical heat generation.

The term "chronic wound" in the context of the present specification particularly refers to a wound that is characterized by an abnormal healing process, e.g., that does not heal in an orderly set of stages and in a predictable amount of time. Particularly, a wound that does not heal within 3 months is considered chronic.

The term "biocompatible" in the context of the present specification particularly refers to the qualities non-toxic and/or non-immunogenic of the magnetic particles.

The term "plurality" in the context of the present specification particularly refers to at least 10, 100, 1,000 or 10,000 magnetic particles.

In certain embodiments, the composition of the invention is provided for application in an alternating magnetic field. In certain embodiments, the alternating magnetic field is repeatedly applied in time intervals between one and fourteen days typically during a time period of six weeks. In defined cases, e.g. chronic diabetes with wound healing impairments, the repeated treatment sessions can be applied for even longer periods of time as an adjunct therapy.

In certain embodiments, the magnetic particles consist of or comprise a ferrimagnetic material, a ferromagnetic material or a superparamagnetic material.

In certain embodiments, the ferrimagnetic material is selected from a ferrimagnetic iron oxide, such as, for example, F₃O₄ (magnetite) or y-Fe₂O₃ (maghemite) or a mixtures thereof, and a ferrite. Advantageously, iron oxide is biocompatible and only slowly biodegradable.

Method and means for manufacturing magnetic particles as described above are known the skilled person. For example, magnetic particles comprising magnetite and/or maghemite may be manufactured by co-precipitation of aqueous Fe²⁺/Fe³⁺ salt solutions by increasing the pH, particularly by addition of a base. The size, shape and composition of the magnetic particles may be controlled by the selection of the salts (for example chloride, sulfates or nitrates), the ratio between Fe²⁺ and F³⁺ (e.g. a Fe²⁺/F³⁺ of 2:1), the reaction temperature, the pH value and the ionic strength of the reaction medium as well adding speed or mixing time at which the base is added to the solution.

In certain embodiments, the ferrite is selected from manganese-zinc-ferrite (MnₐZn₍₁₋ₐ₎Fe₂O₄), nickel-zinc ferrite (NiₐZn₍₁₋ₐ₎Fe₂O₄), and cobalt ferrite (CoFe₂O₄; CoO*Fe₂O₃).

In certain embodiments, the magnetic particles are characterized by a mean size in the range of 10 nm to 100 nm. In certain embodiments, the magnetic particles are assembled into one or more clusters, beads or seeds in the range of 100 µm to 0.5 mm. Particularly, the magnetic particles are characterized by a narrow size distribution e.g. by a full width at half maximum not more then the size value at maximum, or not more than 80%, 50% or 30% of the size value at maximum. Particularly, the plurality of magnetic particles may also comprise magnetic particles that are characterized by a size not more than 50 %, 40% or 30% larger or smaller than the above mentioned mean size. For example, a plurality of magnetic particles characterized by a mean size of 10 nm may accordingly comprise magnetic particles in the range of 5 nm to 15 nm, or in the range of 6 nm to 14 nm, or in the range of 7 nm to 13 nm.

In certain embodiments, the magnetic particles comprise a magnetic core and a coating or a shell, wherein the magnetic core of the particles is characterized by a mean size in the range of 10 nm to 100 nm.

The term "mean size" with regard to the magnetic particles particularly refers to the arithmetic mean or to the median of the size distribution of the magnetic particles. Such mean size may be determined by methods known to the skilled person such as, for example, by static or dynamic light scattering (SLS, DLS) or size-exclusion chromatography.

In certain embodiments, the magnetic particles comprise a coating or a shell such as a layer of silica.

In certain embodiments, the coating consists of or comprises dextran or a carboxylate such as citrate or succinate.

In certain embodiments, the composition of the invention further comprises an antiseptic agent.

The term "antiseptic agent" in the context of the present specification refers to an agent that reduces the possibility of infection, sepsis or putrefaction and particularly has antimicrobial and/or antifungal properties.

In certain embodiments, the antiseptic agent is triclosan (CAS NO 3380-34-5).

In certain embodiments, the composition of the invention further comprises a wound healing agent.

The term "wound healing agent" in the context of the present specification refers to an agent that is able to promote the wound healing, particularly an agent that promotes cell migration and proliferation of endothelial cells and angiogenesis, such as, for example, a collegenase, a Zinc-dependent metalloproteinase, a plasminogen activator or a suitable growth factor.

In certain embodiments, the wound healing agent is selected from
- a growth factor such as the vascular endothelial growth factor (VEGF), the epidermal growth factor (EGF), the platelet derived growth factor (PDGF), the fibroblast growth factor (FGF), the insulin-like growth factor (IGF-1), the human growth hormone, the granulocyte-macrophage colony stimulating factor (GM-CSF) or other,
- a vitamin (A, C, E and others),
- a mineral supplement (zinc, copper and others),
- an antiseptic substance, or
- chitin.

In certain embodiments, the wound healing agent is selected from the available medicines to promote wound angiogenesis and healing.

According to a further aspect of the invention, a dosage for use in a method for wound treatment or wound healing, particularly of a chronic wound, is provided. The dosage form comprises the composition of the invention and optionally a pharmaceutically acceptable excipient.

In certain embodiments, the composition of the invention is applied as a topical formulation. A topical formulation may be, for example, an ointment or dermal patch, or an injectable formulation.

In certain embodiments, the dosage form is provided for application in an alternating magnetic field. Particularly, the magnetic field may be repeatedly applied in time intervals between one and fourteen days typically during a time period of six weeks. In defined cases, e.g. chronic diabetes with wound healing impairments, the repeated treatment sessions can be applied for even longer periods of time as an adjunct therapy.

According to another aspect of the invention, a medical material for wound treatment or wound healing, particularly of a chronic wound, is provided.

The medical material comprises:
- a carrier, and
- the composition of the invention, wherein the composition is attached to or comprised within the carrier.

Also, the medical material of the invention is characterized by several advantages for wound treatment:
- the medical material is simply usable by delivering the material to the wound and applying an alternating magnetic field;
- the carrier is not limited to any specific shape, thus can be designed, for example, as wound dressing, bandage, suture material any many more;
- the medical material may be enhanced by incorporation of wound healing agents, which are particularly embedded in the carrier;
- the medical material allows a simple mechanical removal of the composition of the invention;
- the medical material may used to deliver the composition of the invention to the wound region;
- the medical material may be advantageously used to cover external wound such that a wet environment around the wound can be created that is beneficial for wound healing.

In certain embodiments, the composition is embedded within the carrier. In certain embodiments, the composition is controllably releasable from said carrier.

In certain embodiments, the medical material is provided for application in an alternating magnetic field. Particularly, the magnetic field may be repeatedly applied in time intervals between one and fourteen days typically during a time period of six weeks. In defined cases, e.g. chronic diabetes with wound healing impairments, the repeated treatment sessions can be applied for even longer periods of time as an adjunct therapy.

In certain embodiments, the carrier consists of or comprises a thermo stable material that particularly essentially maintains is chemical and/or physical properties within a temperature range of 30° C to 55 °C, particularly of 30 C to 46 °C, and particularly such that the composition of the invention is kept at or within the carrier at or above a temperature in the range of 30° C to 55 °C. In particular, preferred are temperatures around 46 °C.

In certain embodiments, the thermo stable material is selected from silk, cotton, polyurethane, carboxymethyl cellulose, polyurethane and alginate.

In certain embodiments, the carrier consists of or comprises a thermoresponsive material that particularly changes its chemical and/or physical properties above a temperature in the range of 30°C to 46°C, particularly such that the composition of the invention attached to or comprised within the carrier is released from the carrier at a temperature in the range of 30°C to 46°C.

In certain embodiments, the thermoresponsive material is selected from poly(N-isopropylacrylamide, poly[2-(dimethylamino)ethyl methacrylate], poly(vinylcaprolactame), polyvinyl methyl ether and hydroxypropylcellulose.

In certain embodiments, the carrier is formed by or comprises a hydrogel comprising poly(N-isopropylacrylamide and water or an aqueous solution, wherein the composition is solved or suspended in the water or the aqueous solution. Particularly, such hydrogel will undergo a phase transition above a temperature of 32°C such that the water or the aqueous solution including the composition is released from the hydrogel above 32°C. Advantageously, the release of the composition can be simply triggered by heat, particularly by heat generated upon applying an alternating magnetic field to the composition.

In certain embodiments, the medical material further comprises a means for enhancing the mechanical stability of said medical material, wherein the means is attached to the carrier or comprised within the carrier.

Such means is particularly suitable in case of the chosen material for the carrier exhibits insufficient mechanical stability; for example, in case of the carrier is a hydrogel as described above.

Such means may be designed in form of an additional layer attached to the carrier comprising suitable naturally occurring or artificial fibres such as, for example, fibres made of or comprising polyurethane.

In certain embodiments, the medical material is designed as a suture material, a wound dressing or bandage.

In certain embodiments, the medical material is designed as a wound dressing or a bandage, wherein the wound dressing or the bandage comprises the composition of the invention, particularly the plurality of biocompatible magnetic particles comprised within the composition of the invention, in a concentration of 25 µg per 100 mm³.

In certain embodiments, the medical material is designed as a suture material, a wound dressing or a bandage. Particularly, as suture material, the magnetic material shaped as nanoparticles and comprised within the composition of the invention are intercalated with synthetic or natural organic microfibers.

In certain embodiments, the medical material is designed a wound dressing or a bandage, wherein the wound dressing or the bandage comprises magnetic particles, particularly in form of ferro-, ferri of superparamagnetic iron oxide, in a concentration of 25 µg per 100 mm³.

In certain embodiments, the carrier is or comprises a micropump.

The term micropump in the context of the present specification particularly refers to a pump with functional dimension in the micrometer range, more particularly to a pump that is suitable for the transport of volumes in microlitre or submicrolitre range.

In certain embodiments, the micropump is a micromembrane or microdiaphragm pump comprising a pump membrane or diaphragm and a piezoactuator.

In certain embodiments, the medical material further comprises a means for determining the temperature, the pH value and/or the occurrence and/or the concentration of a compound associated with wound developments or wound healing, such as, for example, lactate.

According to yet another aspect of the invention, a method for wound treatment or wound healing, particularly of a chronic wound, is provided, comprising
- administrating the composition of the invention to a patient in need thereof, particularly to the wound, and
- applying an alternating magnetic field to the composition administered to the patient.

In certain embodiments, the application of the alternating magnetic field to the composition administered to the patient is repeated, particularly until the wound is healed. Typically, the time interval between two heating sessions is 1 to 7 days in order to avoid preconditioning. In certain embodiments, the alternating magnetic field is repeatedly applied in time intervals between one and fourteen days typically during a time period of six weeks. In defined cases, e.g. chronic diabetes with wound healing impairments, the repeated treatment sessions can be applied for even longer periods of time as an adjunct therapy.

In certain embodiments, the alternating magnetic field is characterized by a magnetic field strength (H) in the range of approximately 8 to 40 kA/m, particularly of 15.4 kA/m, and/or a frequency in the range of 400 MHz to 500 MHz, particularly 435 MHz.

In certain embodiments, the composition is administered in form of the medical material of the invention, particularly in form of a wound dressing, a bandage or a suture material.

The invention is further illustrated by the following examples and figures, from which further embodiments and advantages can be drawn. These examples are meant to illustrate the invention but not to limit its scope.

### Description of the figures

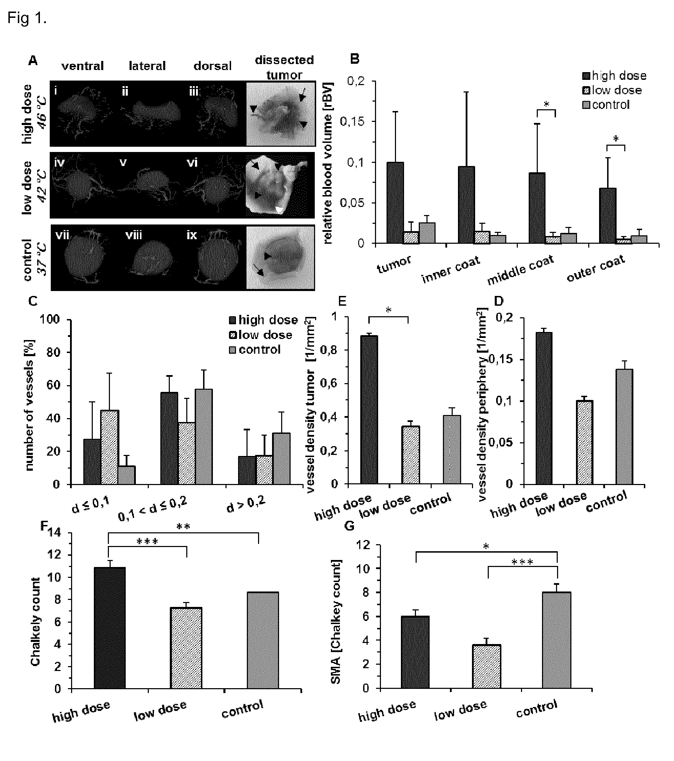

- Fig. 1: shows that hyperthermia influences relative blood volume (rBV) and relative blood vessel density (rBVD) within the tumour and its immediate environment. A) Representative pictures of reconstructed µCT-images showing vasculature (red), green: tumour tissue, arrow: skin, arrowheads: tumour. B) Relative blood volume as estimated by the ratio of vascular volume of three standardized areas that surround the tumour and the tumour itself (for details see Methods). C) Distribution of vessel diameters within the tumour. The number of vessels was related to the tumour volume. D) Vessel density of the tumour. Values were related to the cross sectional area of the tumour. D) Vessel density of the tumour. E) Vessel density of the tumor periphery. The values were related to the cross sectional area of the periphery. F) Quantitative analysis of immunohistochemical staining of CD31 by Chalkley Count. Data are expressed as means +/- SEM. Animals were treated either with low dose (42 °C for 1 h) or high dose hyperthermia (46 °C for 1 h). Controls were not subjected to any hyperthermia treatment. All data refer to 8 days post hyperthermia.

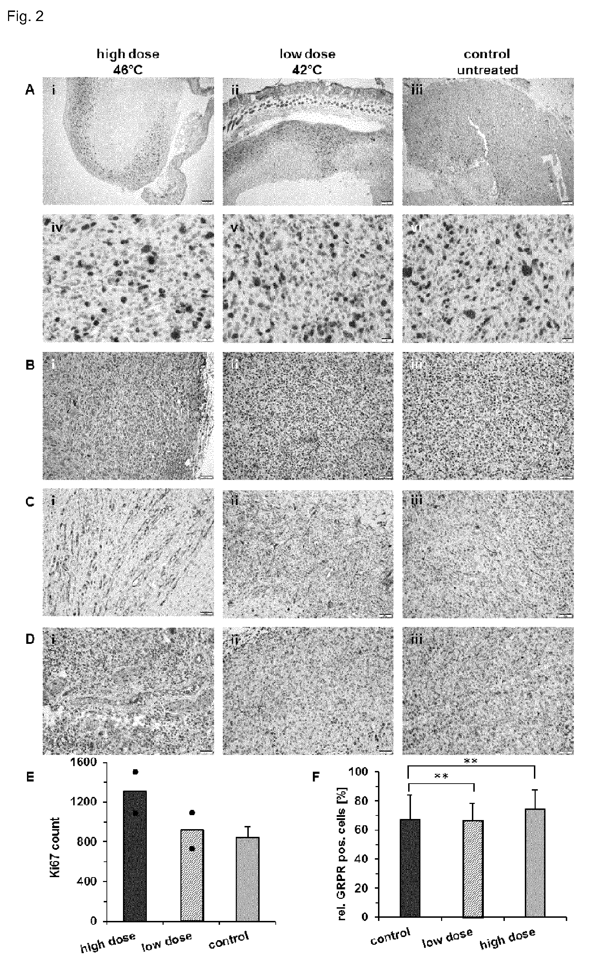

- Fig. 2: shows the effect of hyperthermia on microvessel density, α-SMA, Ki67 and GRPR expression A) Representative images of immunohistochemical staining of Ki67. Used magnification: x 40 fold (i-iii) and x 400 fold (iv-vi). B) Representative images of immunohistochemical staining of GRPR. Used magnification: x 200 fold C) Representative images of immunohistochemical staining of CD31, magnification: 200 fold. D) Representative images of immunohistochemical staining of α-SMA, used magnification: x 200 fold. E) Quantitative analysis of immunohistochemical staining of proliferation marker Ki67. F) Quantitative analysis of immunohistochemical staining of CD31 by Chalkley Count (**: p ≤ 0.01). Animals were treated either with low dose hyperthermia, 42 °C for 1h or high dose hyperthermia, 46 C° for 1 h. Controls were not subjected to any hyperthermia treatment. All data refer to 8 days post hyperthermia.

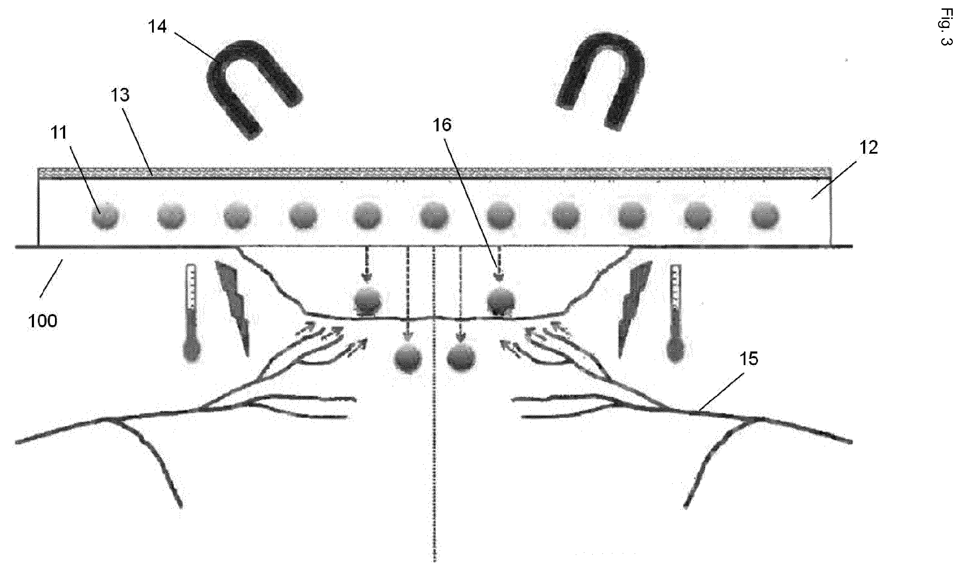

- Fig. 3: shows a schematic drawing of a specific embodiment of the medical material of the invention.

### Examples

The present invention is based on the novel concept of the use of magnetic hyperthermia in wound healing. Accordingly, medical materials 100 comprising magnetic materials 11 that are embedded in a biological carrier 12 are provided by the invention. The carrier 12 may be, for example, a wound dressing, a bandage or a suture material.

Advantageously, iron oxide (magnetite and/or maghemite) are used as magnetic material 11 due to its biocompatibility and degradability, preferably in form of particles 11. Release of heat is facilitated by exposition of the carrier to an alternating magnetic field. The released heat promoted the neoangiogenesis, particularly via the heat stimulated release of VEGF from cells associated with the wound. The embedded magnetic iron oxide material or particles 11 may remain in the carrier 11 or may be released and transported into the wound region 15. This selective process may be achieved by use of thermo stable or thermoresponsive materials 12, in which the magnetic material or particles 11 are embedded.

Advantageously, since iron oxide 11 is inert and is only slowly degraded in the body, multiple sessions for physical stimulation (by applying an alternating magnetic field) of neoangiogenesis are possible.

The medical material 100 may be applied to external wounds on the surface of the body, particularly in form of a wound dressing, or to internal wounds, such as for example, wounds of internal organs, in form of an implant. In form of a suture material, the medical material 100 may be used for treatment of internal or external wounds. Additionally, the medical material 100 may be used for patient suffering chronic wound such as, for example, in the case of diabetes.

### Example 1: Promotion of vascularisation by heat

### Animals and tumour model

All experiments were in accordance with international guidelines on the ethical use of animals and were approved by the regional animal care committee. During experimentation, the animals were anesthetized with 2.0 % - 2.5 % Isoflurane (Actavis). For tumor implantation, 2x10⁶ MDA-MB-231 cells were resuspended in 100 µl Matrigel™ (BD Biosciences) and implanted subcutaneously in the middle of the back of the animals (n= 24).

### External Hyperthermia of tumours in mice

Animals were randomly divided into 3 groups with 8 individuals each: high dose and low dose hyperthermia and non-treated controls. *In vivo* external hyperthermia treatments were performed via exposure of tumours to hot water. During high dose hyperthermia, temperatures of 46 °C were reached at the tumor surface and at least 44 °C in the inner tumor (group 1); during low dose hyperthermia the tumor surface exhibited a temperature of 42 °C, and minimum of 40 °C in the inner tumor tissue. The hyperthermia treatment was conducted for 1 hour. The tumor and body temperature were monitored using fiber-optic thermocouples (OPTOCON AG). These preconditions were determined in a prototype experiment. Tumor size was measured 24 h, 48 h, and 8 days after hot water hyperthermia. The tumor volume was calculated by the formula V=π / 6 x length x width x height of the tumor (Dethlefsen et al., J Natl Cancer Inst. 1968 Feb;40(2):389-405).

### In vivo imaging of GRPR and tumour vascularisation after external hyperthermia.

The intravenous injection of 1 nmol IRDye 800CW RGD and 2 nmol BombesinRSense 680 per mouse was done simultaneously at 24 h and 7 days after treatment to image change of tumor vascularisation, probe accessibility after treatment and GRPR expression. 24 h after administration of the optical probes the fluorescence intensities were monitored contemporaneously by Maestro™ system (CRi Inc.) using the filter "Deep Red" (excitation filter: 670-710 nm, emission filter: 750 nm) for IRDye 800CW RGD, and the filter "Yellow" (excitation filter: 570-610 nm, emission filter: 645 nm) for BombesinRSense 680. The fluorescence intensities for each optical probe and point of time after hyperthermia were related to the native images.

The contrast for each animal was determined by the tumour-to-muscle-ratio (TMR). The mean values of treated animals were related to untreated ones. Contrast agent accumulation was compared to the non-treated control group (n=10).

### Analysis of tumour vascularization via µCT.

Immediately after immolation the mice were prepared for vessel analysis. In this context, the mice were perfused with 10 ml of the lead-based silicon rubber casting resin Microfil® MV-130 and MV-diluent, prepared according to the manufactors guidelines (Flow Tech). All scans were performed using a dual source µCT cone-beam scanner TomoScope® Synergy (CT Imaging GmbH). Scans were visualized and analysed using Imalytics® preclinical software (Philips Research) after three-dimensional volume rendering of reconstructed high-resolution µCT data sets. In order to analyse also the tumor-associated vessels located in the immediate periphery of the tumor, we defined three consecutive areas over the tumour surface, whereas each coat has a defined width of 0.592 mm (8 voxel). The total analysed area surface was of 1.776 mm (24 voxel). The volumes of the respective vessels of each area were calculated in proportion to the entire tumour volume. To calculate the relative blood vessel density (rBVD), the number of vessels in a defined field-of-view (FOV) was counted. Therefore, three randomly chosen digital slices in each tumour were evaluated. The borders of the tumor and of the periphery were chosen to define the different FOV in each of the three planes (Supplement 1 C). Therefore, each FOV was relative to the respective tumor size.

### Hyperthermia affects tumour vascularisation in a temperature dependent manner

Ex vivo µCT imaging showed that the relative blood volume (rBV) in the tumour and its periphery, as an indicator for the vascularisation grade, increased distinctly after high dose hyperthermia (8 days post hyperthermia, Fig. 1A and B). Additionally, both treated hyperthermia groups (high and low dose) showed a distance-specific decrease of the rBV value from the tumor surface to its periphery. This pattern was not detectable in the control group. The increase of the rBV after high dose hyperthermia was accompanied by a change in the number of vessels and the distribution of their vessel diameter (Fig. 1 C). In particular, the number of vessels with diameters larger than d > 0.2 mm decreased distinctly in the hyperthermia treated groups compared to the control ones. Furthermore, vessels with a small diameter increased. Especially low dose hyperthermia treated tumours exhibited mainly small vessels. Interestingly, the expression of smooth muscle actin, as indicator for the presence of mature endothelial cells, decreased after (high and low dose) hyperthermia (Fig. 1 G).

*Ex vivo* µCT imaging indicate that tumors treated with high dose hyperthermia seem to have an advanced blood supply as indicated by a higher relative blood vessel volume (rBV) and an increased blood vessel density (rBVD) at the tumor margin and periphery, and an increased vessel count compared to animals treated with low dose hyperthermia. These are indications for the induction of pro-angiogenic processes, as a result of the release of pro-angiogenic factors from residual tumor cells after the hyperthermic stress (see above). The release of pro-angiogenic factors as a result of hypoxia is a common process in normal tissue. In our case it is artificially induced via magnetic hyperthermia. The reduced number of large vessels and the increase amount of small ones in the hyperthermic treated groups evidence that new blood vessels are formed. This assumption was further verified by the reduced expression of smooth muscle actin (α-SMA), a feature of mature blood vessels. Accordingly, there is also evidence that tumor-associated endothelial cells treated at temperatures up to 47 °C might contribute to an increased invasiveness of corresponding tumors cells. The data supplement the fact that, under defined conditions, hyperthermia affects tumour vascularity, re-growth, invasion and metastatic potential.

After high dose hyperthermia, the tumour tissue exhibited high GRPR expression and high vascularisation particularly at the tumour margin and surroundings. With consideration of the increased of blood volume and the increased expression of GRPR, which mediates mitogenic and morphogenic effects, such residual tumour tissue has the potential for an regrow as it has been described for residual hepatocellular carcinoma. Accordingly, the increased GRPR expression was shown to co-activate EGFR and VEGF. Furthermore, it was found, that the presence of GRP and GRPR improves vascular endothelial cell proliferation and tubule formation by suppressing endothelial cell autophagy via activation of the Pl3K/AKT/mTOR pathway. As well as GRP antagonists in combination with EGFR antagoists effect a decrease in the level of protein expression of VEGF (Kanashiro et al 2007).

***Immunohistochemistry corroborated the in vivo findings after hyperthermia treatment*** The semi-quantitative analysis of immunohistochemical staining of CD31 to calculate microvessel density (Fig. 2B) by Chalkley count showed a significant (p≤ 0.05) increase of tumoural vascularisation after high dose hyperthermia. Additionally, vessel structures with a wider lumen were detected in certain areas in tumours treated with high dose hyperthermia (Fig. 2C, i) compared to those treated with low dose hyperthermia or the non-treated controls. Low dose hyperthermia resulted in a lower vessel density. α-SMA was highest in tumours of the control group (Fig. 1 G) but evenly distributed (Fig. 6D). After High-dose hyperthermia α-SMA staining was diminished except for a few areas, which exhibited large blood vessels with strong staining (Fig. 2D, i).

### Conclusion

It could be shown that hyperthermia with high temperatures induces *in vivo* overexpression of αᵥβ3 integrin and GRPR and increases intratumoural blood vessel volume in residual tumour tissue. Consequently this effect can be advantageously exploited in the treatment of wounds, wherein the re-vascularisation is induced by heat advantageously generated by magnetic particles in an alternating magnetic field.

### Example 2: Magnetic medical material for wound healing

Fig 2 depicts a preferred embodiment of the medical material 100 comprising magnetic particles 11. The medial material 100 is designed a flat wound dressing 100 comprising a heat generating layer, in which the magnetic particles 11 in form of clusters of iron oxide crystals with a mean size of approx. 100 µm are embedded in a thermoresponsive carrier 12, such as, for example, poly(N-isopropy)lacrylamide. The particles 11 may be coated with an organic layer (polyethylene glycol, dextran, starch, hyaluronic acid, chitosan and the like) to prevent aggregation of the particles 11 within the carrier 12. Alternatively, the particles 11 are coated with silica. Furthermore, the medical material may comprise up to 90 % (w/w) magnetic material or particles 11. After administration of the medical material 100 to a wound of a patient, an alternating magnetic field 14 is applied to the magnetic particles 11, which then release heat to the wound promoting particularly the neoangiogenesis of vessels 15 located at or in the vicinity of the wound, particularly by triggering the release of VEFG by cell located at or in the vicinity of the wound. Due to the nature of the thermoresponsive carrier 12, the magnetic particles 11 are released 16 into wound during or after heat release by the particles 11.

Principally, the amount of the magnetic material 11 is dependent of parameters of the alternating magnetic field 15 used for the treatment of the wound. For example, an amount of 25 µg magnetic iron oxide per mm³ medical material is sufficient to provide an increase in temperature from 37° C to 43° C during exposure of an alternating magnetic field with a field strength typically of approx. 15.4 kA/m and a frequency of approx. 435 MHz.

Alternatively, the medical material 100 may be designed to keep the magnetic particles 11 within the carrier 12. In this case the carrier would be formed by thermo stable materials such as silk, alginate, polyurethane, carboxymethyl cellulose, cotton, or the like.

The medical material 100 or the carrier 12 itself may further comprise mechanically stabilizing material 13 enhancing the structural integrity or flexibility such as for example polyurethane. Such mechanically stabilizing material 13 may be comprised with the medical material in form of a separate layer 13 attached to the above-named heat generating layer 12.

Generally, this separate layer 13 may be formed by any suitable artificial or naturally occurring material such as fibres and comprises a adhesive matrix for attaching the separate layer to the heat generating layer.

Advantageously, the carrier 12 or the whole medical material 100 is biocompatible and biologically degradable, particularly in case of the medical material 100 is used as a pad or implant (typically iron oxide particles imbedded in ceramics, hydroxylapatite, calcium phosphates supplementes which could also be supplemented with matrix materials for cells (such as collagens, elastins etc.) or as a suture material (typically particles intercalated in silk fibres, chitosan, collagen, polyamide, polyethylene, polypropylene, terephthalate, polyactic acid, polylactic acid, polyglycolic acid, poly(lactide-co-glycolide) and derivatives, poly (ε-caprolactone), polyp-dioxanone, poly(trimethylene carbonates), polyhydroxyalkanoates, and others) for internal wounds.

The carrier 12 or the medical material 100 may further comprises antiseptic substances such as triclosan, and optionally biologically active agents such as a growth factor.

With regards to the desired function the medical material 100 or the carrier 12 may be designed flat or spherical, or stiff or flexible.

The magnetite particles may be manufactured as described in Andrade et al. (Quim. Navo, Vol. 33, No 3. 524-527, 2019) or in Laurent et al. (Chem. Rev. 2008, 108, 2064-2110).

## Claims

1. A composition for use in a method for wound treatment, particularly of a chronic wound, particularly by magnetic hyperthermia, **characterized in that** said composition (11) comprises a plurality of biocompatible magnetic particles (11).

2. The composition for use in a method for wound treatment according to claim 1, **characterized in that** said composition (11) is applied in an alternating magnetic field.

3. The composition for use in a method for wound treatment according to claim 1 or 2, **characterized in that** said magnetic particles (11) comprise or consist of ferrimagnetic, ferromagnetic or superparamagnetic iron oxide, particularly Fe₃O₄ and/or γ-Fe₂O₃, or a ferrite, particularly a manganese-zinc-ferrite, a nickel-zinc ferrite or a cobalt ferrite.

4. The composition for use in a method for wound treatment according any one of the preceding claims, **characterized in that** said magnetic particles (11) are **characterized by** a mean size in the range 10 nm to 100 nm.

5. The composition for use in a method for wound treatment according any one of the preceding claims, **characterized in that** said magnetic particles (11) comprise a magnetic core and a coating, wherein said magnetic core is characterized a mean size in the range of 10 nm to 100 nm.

6. The composition for use in a method for wound treatment according to any one of the preceding claims, **characterized in that** said magnetic particles (11) are assembled into one or more clusters with a mean size in the range of 100 µm to 500 µm.

7. The composition for use in a method for wound treatment according to any one of the preceding claims, **characterized in that** said composition (11) further comprises an antiseptic agent, particularly triclosan.

8. The composition for use in a method for wound treatment according to any one of the preceding claims, **characterized in that** said composition (11) further comprises a wound healing agent, particularly a growth factor such as the vascular endothelial growth factor, the epidermal growth factor, the platelet derived growth factor, the fibroblast growth factor, the insulin-like growth factor, the human growth hormone, the granulocyte-macrophage colony stimulating factor, or a vitamin, particularly vitamin A, vitamin C or vitamin E, a mineral supplement, particularly zinc or copper, or chitin.

9. A dosage form for use in a method for wound treatment, particularly of a chronic wound, comprising a composition (11) according to any one of the preceding claims, and optionally a pharmaceutically acceptable excipient, wherein particularly said composition is applied as a topical formulation.

10. The dosage form for use in a method for wound treatment according to claim 9, **characterized in that** said dosage form is applied in an alternating magnetic field.

11. Medical material (100) for wound treatment, particularly of a chronic wound, comprising.
- a carrier (12), and
- a composition (11) according to any one of claims 1 to 8, wherein said composition is attached to or comprised within said carrier, particularly embedded within said carrier (12).

12. The medical material according to claim 11, **characterized in that** said carrier (12) consists of or comprises a thermo stable material that maintains its chemical and/or physical properties within temperature range of 30°C to 46°C, particularly such said composition (11) remains attached to or comprised within said carrier (12), wherein particularly said thermo stable material is selected from silk, alginate, polyurethane, carboxymethyl, cellulose and cotton.

13. The medical material according to claim 11, **characterized in that** said carrier (12) consists of or comprises a thermoresponsive material that changes its chemical and/or physical properties at a temperature in the range of 30°C to 46°C, particularly such that said composition (11) is released from said carrier (12), wherein particularly said thermoresponsive material is selected from poly(N-isopropy)lacrylamide, poly[2-(dimethylamino)ethyl methacrylate], poly(vinylcaprolactame), polyvinyl methyl ether and hydroxypropylcellulose.

14. The medical material according to any one of claims 11 to 13, **characterized in that** said medical material (100) further comprises a means (13) for enhancing the mechanical stability of said medical material (100), wherein said means is attached to said carrier (12), particularly in form of an addition layer (13), or comprised within said carrier (12), and wherein particularly said means (13) consists of or comprises a naturally occurring or artificial fibre.

15. The medical material according to any one of claims 11 to 14, **characterized in that** said medial material (100) is designed as a suture material, a wound dressing or bandage, wherein particularly said medical material (100) is designed as a wound dressing or a bandage, and said wound dressing or said bandage comprises said composition (11) in a concentration of 25 µg per 100 mm³.
